# EUROPEAN PATENT SPECIFICATION

(11) **EP 1 542 903 B1**
(45) Date of publication and mention of the grant of the patent: **20.05.2015**
(21) Application number: 03748966.3
(22) Date of filing: 22.07.2003
(51) Int. Cl.: A61J 3/07, A61K 8/02, B65B 25/06, A61Q 11/00, B65B 11/50, A61K 9/70

(54) **PACKAGING AND DISPENSING OF RAPID DISSOLVE DOSAGE FORM**
VERPACKUNG UND VERABREICHUNG EINER SCHNELL AUFLÖSENDEN DOSIERFORM
EMBALLAGE ET DISTRIBUTION D'UNE FORME POSOLOGIQUE A DISSOLUTION RAPIDE

(30) Priority: 22.07.2002 US 397703 P
(43) Date of publication of application: 22.06.2005
(73) Proprietor: MonoSolRX, LLC, Portage IN 46368 (US)
(72) Inventor: YANG, Robert, K., Flushing, NY 11355 (US); FUISZ, Richard, C., McLean, VA 22101 (US); MYERS, Gary, L., Kingsport, TN 37660 (US); FUISZ, Joseph, M., Washington, D.C. 20037 (US)
(74) Representative: EP&C
(86) International application number: PCT/US2003/022882
(87) International publication number: WO 2004/009445

(56) References cited:
- WO-A-00/42992
- WO-A-01/68452
- WO-A-01/70194
- US-A- 4 029 757
- US-A- 4 849 246
- US-A- 4 925 670
- US-A- 5 806 284
- US-A- 5 948 430
- US-B1- 6 287 595
- US-B1- 6 655 112
- US-B2- 6 656 493

## Description

### FIELD OF THE INVENTION

The present invention relates to devices and methods for the storage and dispensing of an edible, thin, water-soluble, rapid dissolve dosage form.

### BACKGROUND OF RELATED TECHNOLOGY

Water-soluble thin films have recently become very popular as a form of breath freshener. These films generally include a breath freshening agent in a polymer film. These films have a convenient small size which contributes to their popularity.

However, such films and their current packaging have several disadvantages. The films themselves are typically too thin to support other active ingredients, such as a pharmaceutical active. In addition, the packaging does not provide an effective air/moisture barrier. The result is a film that frequently dries out, becoming too brittle for use. Furthermore, when the films are placed in the packaging, they are usually stacked, and frequently adhere to an adjacent film. Therefore, a person attempting to remove a single film from the packaging may inadvertently remove two or more.

It is desirable to provide a film and packaging that includes a barrier to moisture, air, and light, which can interfere with the quality of the film product and the active ingredients contained within the film. Ideally, this film will be capable of supporting not only a breath freshener as the active, but also pharmaceutical products. It is further desirable to provide a method of dispensing the films, wherein only the desired number of films may be removed at a time.

Documents US-A-4 029 757 and WO 00/42992 each disclose a dosage form. US-B1-6 287-595 discloses a system and method for providing a product in a sealed package. US-A-5 806 284 discloses a system for packaging a water soluble medicament film.

Although US-A-4 029 757 states that its web may be cast into a thin sheet.

US-A-4 029 757 is completely silent with respect to its active varying no more than 10% among dosage units, and provides no disclosure with respect to how to make such a cast edible film having no more than 10% variance of active among dosage units.

With regard to WO 00/42992, this document discloses a strip of film which has been cut into single dose film units. As such, there is no disclosure or suggestion in WO 00/42992 of weakened sections which divide film into segments that represent individual dosage units. US-B1-6 287 595 fails to disclose or suggest weakened sections which divide film into segments that represent individual dosage units.

US-A-5 806 284 discloses cutting film into dosage forms using knives. In US-A-5 806 284, the perforations are in the packaging and not in the film itself. As such, US-A-5 806 284 fails to disclose or suggest weakened sections which divide film into segments that represent individual dosage units.

### SUMMARY OF THE INVENTION

The present invention provides an oral dosage delivery vehicle including an edible film having a sheet-like construction, wherein the film comprises dosage units releasably joined by one or more weakened sections, which permit said dosage units to be detached from the film.

Embodiments of the invention are:
1. An oral dosage delivery vehicle comprising at least one layer of a cast edible film including a uniformly distributed active ingredient, wherein said film comprises weakened sections which divide the film into segments that represent individual dosage units, and wherein said active ingredient varies no more than 10% among said dosage units.
2. The delivery vehicle of embodiment 1, wherein said film is self-supporting.
3. The delivery vehicle of embodiment 1, wherein said weakened sections are perforations.
4. The delivery vehicle of embodiment 1, wherein said weakened sections are scored.
5. The delivery vehicle of embodiment 1, wherein said active ingredient is a pharmaceutical or cosmetic active ingredient.
6. The delivery vehicle of embodiment 1, wherein said film includes a carrier layer of a substantially water insoluble material.
7. The delivery vehicle of embodiment 6, wherein said carrier layer is substantially continuous and said carrier layer includes weakened sections that correspond to the weakened sections of said film.
8. The delivery vehicle of embodiment 1, wherein the film has a surface with a surface area comprising voids that increase the surface area.
9. The delivery vehicle of embodiment 8, wherein said voids increase the speed of dissolution of said delivery vehicle.
10. The delivery vehicle of embodiment 8, wherein the voids are selected from the group consisting of voids that go through the entire depth of the film, voids that go through a portion of the depth of the film, and voids that are formed by casting the film on a patterned template.
11. An oral dosage delivery vehicle comprising at least one layer of a cast edible film, wherein said film comprises dosage units releasably joined by one or more weakened sections, which permit said dosage units to be detached from said film, wherein an active ingredient varies no more than 10% among said dosage units.
12. The delivery vehicle of embodiment 11, wherein said weakened sections contain less film composition than surrounding areas.
13. The delivery vehicle of embodiment 10, wherein said film is self-supporting.
14. The delivery vehicle of embodiment 11, wherein said weakened sections are perforations.
15. The delivery vehicle of embodiment 11, wherein said weakened sections are scored.
16. The delivery vehicle of embodiment 11, wherein said weakened sections are thinner than surrounding area.
17. The delivery vehicle of embodiment 12, further comprising an active ingredient, wherein said active ingredient is a pharmaceutical or cosmetic active ingredient and said active ingredient is uniformly distributed.
18. The delivery vehicle of embodiment 11, wherein said film includes a carrier layer of a substantially water insoluble material.
19. The delivery vehicle of embodiment 18, wherein said carrier layer is substantially continuous and said carrier layer includes weakened sections that correspond to the weakened sections of said film
20. The delivery vehicle of embodiment 17, wherein the film has a surface with a surface area comprising voids that increase the surface area.
21. The delivery vehicle of embodiment 20, wherein the voids are selected from the group consisting of voids that go through the entire depth of the film, voids that go through a portion of the depth of the film, and voids that are formed by casting the film on a patterned template.
22. The delivery vehicle of embodiment 20, wherein said voids are uniform and maintain the uniform distribution of the active ingredient.
23. A package for the storage and dispensing of a sheet-like rapid dissolve dosage form, comprising: a) a pouch comprising a top layer and a bottom layer each having an outer edge, wherein said top and bottom layers are sealed at the respective outer edges to define an enclosed interior surface there within, and b) a sheet-like rapid dissolve dosage form contained within said enclosed space wherein said dosage form comprises at least one layer of a cast edible film comprising weakened sections which divide the film into segments that represent individual dosage units, and wherein an active ingredient varies no more than 10% among said dosage units.
24. The package of embodiment 23, wherein said dosage form includes an active ingredient and comprises a sheet of one or more dosage forms.
25. The package of embodiment 23, wherein said weakened sections include perforations.
26. The package of embodiment 23, wherein said weakened sections are thinner than surrounding.
27. The package of embodiment 23, wherein said weakened sections form one or more equally spaced lines in a direction selected from vertical, horizontal, and combinations thereof.
28. The package of embodiment 23, wherein said pouch is resealable.
29. A method of storing a sheet-like rapid dissolve dosage form comprising the steps of: a) preparing a sheet-like dosage form that includes unit doses of an active ingredient; wherein said dosage form comprises at least one layer of a cast edible film comprising weakened sections which divide the film into segments that represent individual dosage units and wherein the active ingredient varies no more than 10% among said dosage units; b) preparing a pouch comprising top and bottom layers each having an outer edge; c) placing said dosage form between said top and bottom layers; and d) sealing the outer edges of said pouch.
30. A method of dispensing a sheet-like dosage form comprising the steps of: a) preparing a sheet-like dosage form that includes an active ingredient; wherein said dosage form comprises at least one layer of a cast edible film comprising weakened sections which divide the film into segments that represent individual dosage units and wherein the active ingredient varies no more than 10% among said dosage units; b) preparing a pouch comprising top and bottom layers each of said layers having an edge surrounding the circumference of said layer; c) placing said dosage form between said top and bottom layers; d) separably sealing a portion of said edges of said pouch; e) opening a portion of said pouch; opening one or more of said sections of said dosage form along said perforations; and g) removing said one or more dosage units from said pouch.
31. The method of embodiment 30, further comprising the step of resealing said pouch.
32. The method of embodiment 30, further comprising the step of permanently sealing the remaining portion of the edges of said pouch.
33. The delivery vehicle of embodiment 1, further comprising an additional layer of an edible film.
34. The delivery vehicle of embodiment 11, further comprising an additional layer of an edible film.
35. A pouch comprising a top layer comprising a laminate of at least two layers and a bottom layer comprising a laminate of at least two layers, wherein said pouch is sealed along its perimeter, and wherein at least one layer of an edible film comprising weakened sections, which divide the film into segments that represent individual dosage units, wherein an active ingredient varies no more than 10% among said dosage units, is included in the pouch.
36. A pouch comprising a top layer and a bottom layer, wherein said pouch is resealable along a portion of its perimeter and wherein at least one layer of an edible film, comprising weakened sections which divide the film into segments that represent individual dosage units, wherein an active ingredient varies no more than 10% among said dosage units, is included in the pouch.
37. The pouch of embodiment 36, wherein a portion of the perimeter is provided with a pressure-sensitive adhesive.
38. The pouch of embodiment 36 wherein a portion of the perimeter may be sealed and resealed by forming a zipper track along the top layer.
39. The pouch of embodiment 38 further comprising a corresponding track along the bottom layer.

### BRIEF DESCRIPTION OF THE DRAWINGS

FIG. 1 - FIG. 3 are perspective views of the sheet-like dosage forms of the present invention.
FIG. 4 is a perspective view of the unassembled top and bottom sheets including the sheet-like dosage form.
FIG. 5 is a perspective view of the assembled packaging of the present invention.
FIG. 6 is a cross-section of a laminate that may be used as the top or bottom layer of the packaging.
FIG. 7 - FIG. 9 and FIG. 9a are cross-sections of the packaging along line 20-20, including a resealable edge.
FIG. 10 is a cross-section of the packaging along line 20-20, showing the inclusion of more than one active-containing sheet
FIG. 11 is a topside view of the dosage form showing details of a weakened section.
FIG. 12 - FIG. 13 are cross-sections of the dosage form along line 50-50, showing, in detail, different configurations of the weakened section.
FIG. 14 is a topside view of the dosage form showing details of a weakened section.
FIG. 15 is a side view of the detail of a weakened section.
FIG. 16 - FIG. 17 are side views of the dosage form including a backing layer.
FIG. 18 is a perspective view of a film that includes a surface altered to increase the surface area.
FIG. 19 - FIG. 21 are cross-sections along line 60-60 showing the detail of the surface alteration.

### DETAILED DESCRIPTION OF THE INVENTION

The present invention includes a "sheet-like" dosage form or a film. For the purposes of this invention the term "sheet-like" dosage form, "sheet," and film are meant to include a water soluble delivery system having a thickness of less than about 15mils. The sheet-like dosage forms are desirably edible and contain an active ingredient.

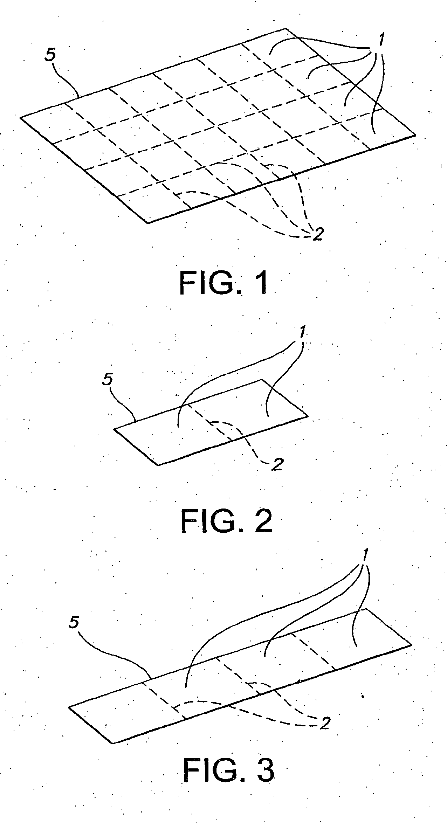

The sheets or films 5 of the water-soluble rapid dissolve dosage forms, which may include one or more dosage forms 1, may include weakened sections, as shown in FIG. 1-3. The weakened section is designed such that application of a bending force thereat breaks apart adjacent units from each other. The weakened sections 2 in the sheet may form straight lines in either a vertical direction or a horizontal direction, or combinations of both, although lines which are not straight may also be used. The weakened sections will divide the sheet into segments that represent individual doses of an active. The segments may be in a variety of different shapes and sizes including square, rectangle, triangle, trapezoid, circle, ellipse, etc. Desirably, the lines will combine to provide sections of substantially equal area. Each section will represent either an individual dosage form or a predetermined fraction of a dosage form. For example, a child dose may be one-half of an adult dose for a particular active, or where a large film is required for a particular dose, the film may be divided to provide ease of administration. For example, in Figure 2, which shows a sheet perforated into two sections, each section may be a dosage unit, or the entire sheet may be a single dosage unit.

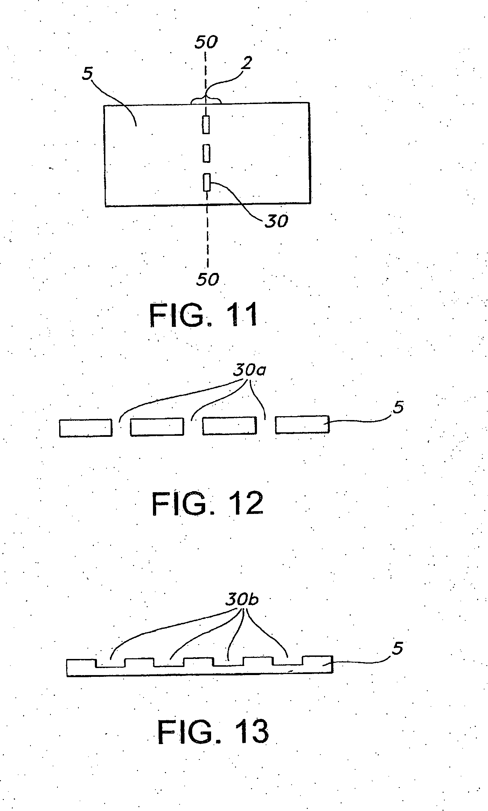

The weakened sections themselves may take on a variety of different configurations, as shown in FIGS. 11-13. In general, a weakened section of the sheet is a location of the sheet that has been altered to permit separation of sections of the film. Desirably, the weakened sections are formed so that the individual dosage units are of substantially identical dimensions. Where the dosage form includes an active, there is uniformity among dosage units that have been separated, desirably where there is less than 10% variance among the individual dosage units both before and after separation. The weakened sections may be in any configuration that permits one section of the film to be separated from the remaining film. Examples of weakened sections include perforations or scored areas that form voids **30** in the material, as in FIG 11. As shown in FIG 12 and FIG 13, the voids 30a and **30b**, respectively, may either completely or partially penetrate the film.

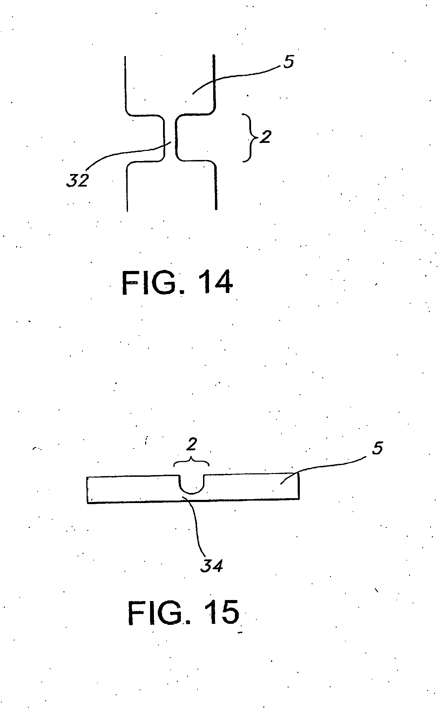

Other examples of weakened sections may be used when uniformity of film size is not an issue, including where the dosage form does not incorporate a drug active. Examples include narrower sections **32** of the film **5**, as shown in FIG. 14, and areas that have less thickness **34** than the surrounding film, as shown in FIG 15. The weakened sections maybe formed by cutting the film, by casting the film into a pre-determined shape, or by casting the film onto a patterned surface that results in specifically selected thinner areas of the film.

The weakened sections, described above, serve as break-points where the film is intended to be separated into individual dosage units. The film itself is generally flexible to avoid inadvertent or premature separation and breaking of dosage units. However, the weakened sections allow the film to be separated at pre-determined segments that will represent the individual doses. This separation may be by breaking, bending, tearing, or otherwise detaching the individual segments of the film or sheet-like construction. The dosage forms may include a line formed of an edible ink along the weakened sections. Such a line serves as a "safety" indication line to visibly indicate to the user that they have broken off the appropriate dosage unit. Particularly where a perforated dosage form is used, this will assure that the user has detached the dosage form at the appropriate location. This may be accomplished by perforating the film with a serrated knife-like instrument, which may include edible ink liners that mark the film as it is cut. Other methods of forming the safety line, such as printing, may be used.

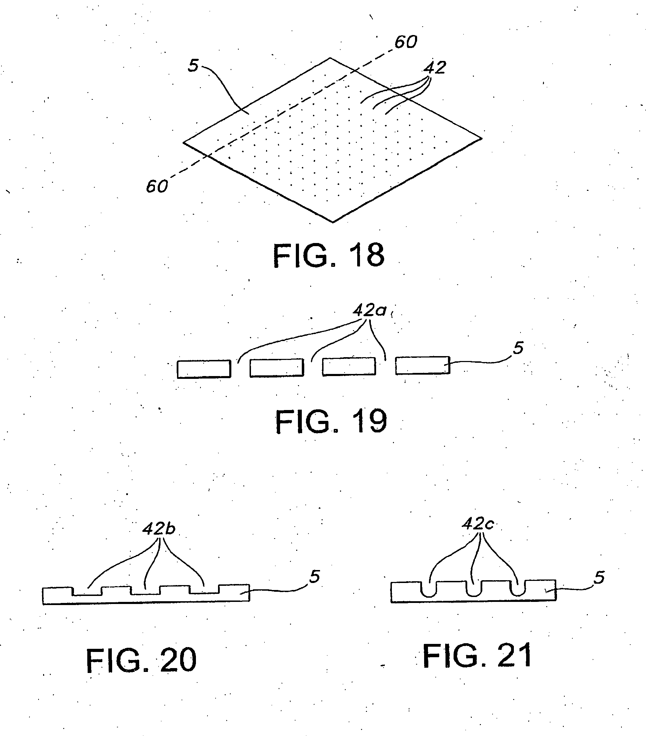

The surface of the film may either be smooth, or altered in a way to increase the surface area of the film. Where the surface area is altered, it may include voids 42 or holes as shown in FIG. 18. The voids will be placed in the film in a uniform manner, which does not affect the uniformity of the distribution of any active that the film may contain. The effect of the voids is an increase in the surface area of the film, which will speed the dissolution time of the film when administered, including an increased speed of dissolution of the film in the mouth. As shown in FIGS. 19-21, the voids may take a variety of different shapes, and may go either completely through the depth of the material as the voids **42a** of FIG. 19 or partially through as the voids **42b** and **42c**, of FIGS. 20 - 21, respectively. The alteration of the surface area of the film will be conducted in such a way that will maintain the strength of the film, unlike the alteration at the weakened sections. Ideally, the voids are formed by either cutting the film or by casting the film on a template to produce a specific pattern.

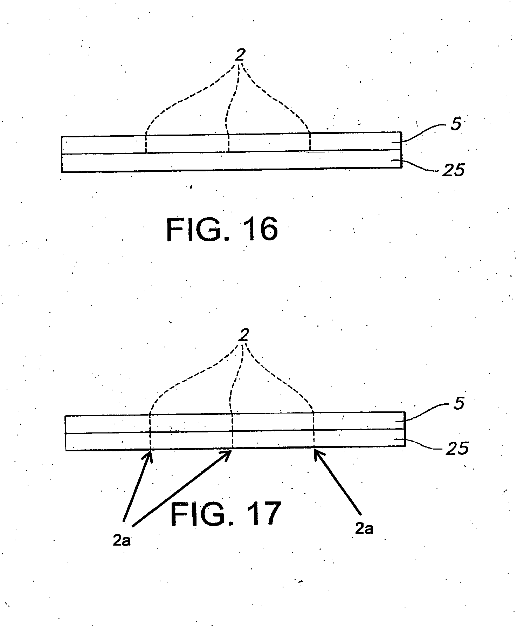

As shown in FIGS. 16 and 17, the sheet-like dosage form **5** may include a substantially water-insoluble backing layer **25.** While the films **5** may be self-supporting, the second carrier layer **25** may act as a support layer for the dosage form **5**. The carrier layer **25** may either be continuous, without the inclusion of weakened sections **2** as in FIG. 16, or it may include weakened sections **2a** corresponding to the weakened sections **2** of the film **5** to allow the second layer **25** to be separated along with a corresponding section of the dosage form **5**, as shown in FIG 17.

The edible sheet-like dosage forms of the present invention include a water-soluble polymer. Useful water-soluble polymers for the present invention include cellulosic materials, gums, proteins, starches, and combinations thereof.

One advantage of the present invention is that dosage units or a portion thereof may easily be dispensed. For example, a 10 mg dosage unit may itself contain a break-point section, i.e., a perforated section, to allow the patient to divide the taking of the required 10 mg dose over two different time intervals, or to simply make it easier to ingest at one time.

Examples of cellulosic materials include, without limitation, carboxymethyl cellulose, hydroxyl methyl cellulose, hydroxyethyl cellulose, hydroxypropyl cellulose hydroxypropylmethyl cellulose, and combinations thereof.

Examples of water-soluble gums include gum arabic, xanthan gum, tragacanth, acacia, carageenan, guar gum, locust bean gum, pectin, alginates and combinations thereof.

Examples of other polymeric materials include polyvinyl alcohol, polyacrylic acid, polyvinyl pyrrolidone, poly(meth)acrylate, poly(meth)copolymers and combinations thereof.

Useful starches include gelatinized, modified or unmodified starches. The source of the starches may vary and include tapioca, rice, corn, potato, wheat and combinations thereof.

Useful water-soluble protein polymers gelatin, zein, gluten, soy protein, soy protein isolate, whey protein, whey protein isolate, casein, levin, collagen and combinations thereof.

Additional water-soluble polymers include dextrin, dextran and combinations thereof, as well as chitin, chitosin or combinations thereof, and polydextrose.

The sheet-like dosage forms of the present invention further include an active component selected from cosmetic agents, pharmaceutical agents, bioactive agents, including antigens, such as ragweed pollen, and combinations thereof. The active component may be present in any amount effective for the intended treatment. It is particularly desirable and an advantage of the present invention that the active component can be included in high loads. For example, the active component may be present in amounts up to about 60% by weight of the total composition and desirably in amounts of 0.01% to about 50% by weight of total composition.

The active components that may be incorporated into the films of the present invention include, without limitation, medicaments, flavors, fragrances, enzymes, preservatives, sweetening agents, colorants, spices, vitamins and combinations thereof.

A wide variety of medicaments and pharmaceutical compositions may be included in the dosage forms of the present invention. Examples of useful drugs include ace-inhibitors, antianginal drugs, anti-arrhythmias, anti-asthmatics, anti-cholesterolemics, analgesics, anesthetics, anti-convulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aides, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

The dosage forms of the present invention further includes one or more members selected from taste-masking agents, plasticizing agents, surfactants, emulsifying agents, thickening agents, binding agents, cooling agents, saliva-stimulating agents, sweetening agents, antimicrobial agents, antigens and combinations thereof.

### Examples

Water soluble thin film compositions Useful in the present invention are prepared using the amounts described in Table 1.

**TABLE 1**

| | **WEIGHT %** | | | | | | | | | |
|---|---|---|---|---|---|---|---|---|---|---|
| **Ingredient** | **A** | **B** | **C** | **D** | **E** | **F** | **G** | **H** | **I** | **J** |
| Hydroxypropylmethyl cellulose | 4.03 | 3.77 | 3.70 | 3.84 | 0 | 3.67 | 4.03 | 0 | 6.24 | 6.24 |
| Peppermint oil | 2.94 | 1.93 | 2.39 | 0 | 0 | 2.67 | 2.94 | 2.67 | 4.17 | 0 |
| Sweetener | 2.20 | 0.32 | 0.23 | 0 | 0.17 | 1.53 | 2.20 | 1.54 | 3.34 | 3.34 |
| Polyvinylpyrrolidone | 2.68 | 2.01 | 2.39 | 0 | 0 | 2.33 | 2.68 | 2.34 | 4.16 | 4.16 |
| Tween 80¹ | 2.24 | 1.07 | 1.48 | 1.42 | 0.55 | 1.35 | 2.24 | 0 | 0 | 0 |
| Simethicone² | 0.66 | 0.42 | 0.68 | 0.22 | 0.22 | 5.00 | 2.00 | 0 | 0.98 | 0.98 |
| Listerine³ | 0 | 0 | 0 | 0 | 92.41 | 0 | 0 | 0 | 0 | 0 |
| Raspberry flavor | 0 | 0 | 0 | 0 | 0 | 0 | 0 | 0 | 0 | 0.12 |
| Cornstarch⁴ | 2.68 | 0 | 0 | 0 | 0 | 0 | 2.68 | 0 | 4.16 | 4.16 |
| Water | 73.53 | 90.47 | 89.14 | 92.22 | 0 | 83.45 | 72.19 | 93.46 | 62.15 | 60.00 |
| Loratadine⁵ | 4.29 | 0 | 0 | 2.31 | 0 | 0 | 4.29 | 0 | 6.65 | 0 |
| Pullulan⁶ | 0 | 0 | 0 | 0 | 6.65 | 0 | 0 | 0 | 0 | 0 |
| Calcium Carbonate | 1.43 | 0 | 0 | 0 | 0 | 0 | 1.43 | 0 | 2.22 | 12.15 |
| Xanthan Gum | 0.30 | 0 | 0 | 0 | 0 | 0 | 0.30 | 0 | 0.46 | 0 |
| Propylene Glycol | 3.02 | 0 | 0 | 0 | 0 | 0 | 3.02 | 0 | 4.67 | 8.84 |
| Ethoxylated castor oil⁷ | 0 | 0 | 0 | 0 | 0 | 0 | 0 | 0 | 0.80 | 0 |

| | | | | | | | | | | |
|---|---|---|---|---|---|---|---|---|---|---|
| ¹Available from ICI Americas ²Available from OSI ³Available from Pfizer, Inc. including thymol (0.064%), eucalyptol (0.092%), methyl salicylate (0.060%), menthol (0.042%), water (up to 72.8%), alcohol (26.9%), benzoic acid, poloxamer 407, sodium benzoate, and caramel color ⁴Available from Grain Processing Corporation as Pure Cote B792 ⁵Available from Schering Corporation as Claritin ⁶Available from Hayashibara Biochemical Laboratories, Inc., Japan ⁷Available as Cremophor EL from BASF | | | | | | | | | | |

The ingredients of inventive compositions A-J were combined by mixing until a uniform mixture was achieved. Vacuum was then applied over 20 min. starting at 500 mmHg and ending at 660 mmHg until all air was removed from the suspension. The compositions were then cast onto a silicone coated paper using a 200 micron spiral wound rod and a K control Coater Model 101 (RK Print Coat Inst. Ltd.). These films were then dried by applying heat at 90°C to the bottom of the film. No external thermal air currents were present above the film. The films were dried to less than about 6% by weight water in about 4 to 6 minutes. The films were flexible, self-supporting and provided a uniform distribution of the components within the film.

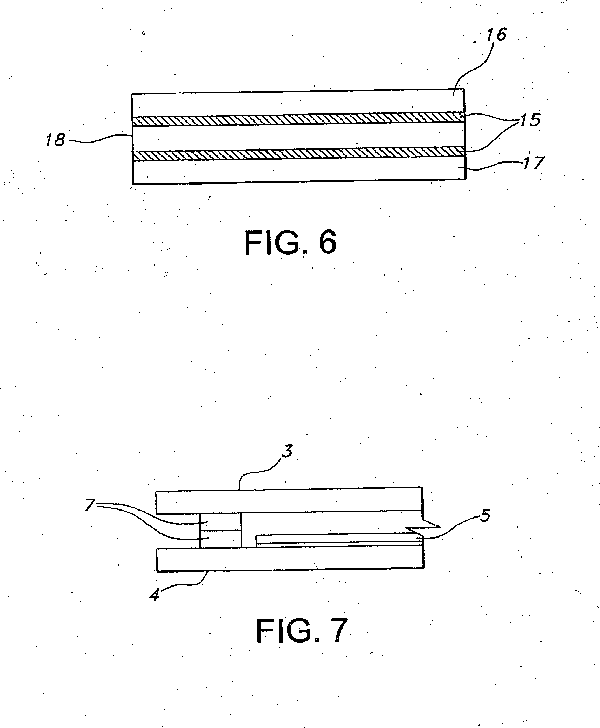

The layers that form the pouch of the present invention maybe made of a variety of different materials and constructions. As shown in FIG. 6, may themselves include one or more layers that are laminated together with an adhesive. A variety of different materials may be used for each of the layers. Desirably, the top and bottom layers will each include a laminate of at least two layers. More desirably, a three-layer laminate will be included. The three layer laminate will include an outer layer **16**, an inner layer **17**, and an intermediate layer **18** that include an adhesive **15** dispersed therebetween.

One effective barrier material is a metal foil, such as aluminum, which provides a barrier to light, moisture and air. Depending on the barrier requirements of the film and any active contained therein, other materials may be selected for the various layers. These materials may include paper, polyolefins, such as polyethylene or polypropylene, polyester, hydrolyzed polyvinyl acetate co-polymer and blends thereof.

The layers may also include, where necessary, an anti-static agent, anti-fogging agent, ultraviolet light absorber, antioxidant, plasticizer, lubricant, nucleating agent, dispersant, colorant, anti-fungus agent, anti-microbial agents, inorganic filler, and the like.

In one aspect of the invention, the layers may be laminated together by first dispersing an adhesive between the layers which may then be co-extruded to form the multilayered packaging of the present invention. The adhesive may include polyolefin resins such as those modified with unsaturated carboxylic acid or a derivative thereof. The unsaturated carboxylic acid may include, without limitation acrylic acid, methacrylic acid, maleic acid, fumaric acid, crotonic acid, itaconic acid, citraconic acid and the like, as well as esters and anhydrides thereof.

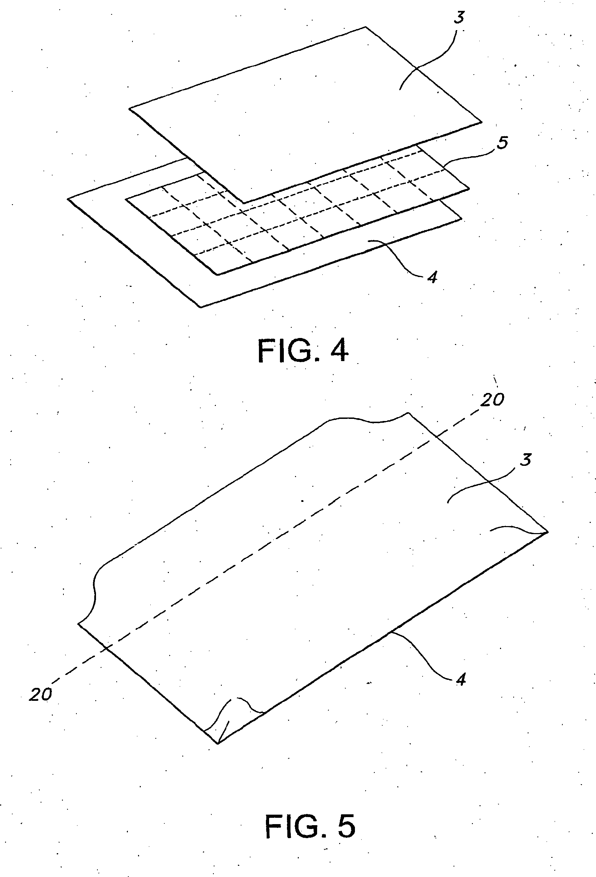

FIG 4 shows layers of a pouch containing the sheet-like dosage forms **5**. The pouch includes top **3** and bottom **4** layers; which may each include a multi-layered laminate material, which is sealed along the perimeter as shown in FIG. 5. This may be accomplished, for example, by heat sealing or with an adhesive, such as a pressure-sensitive adhesive.

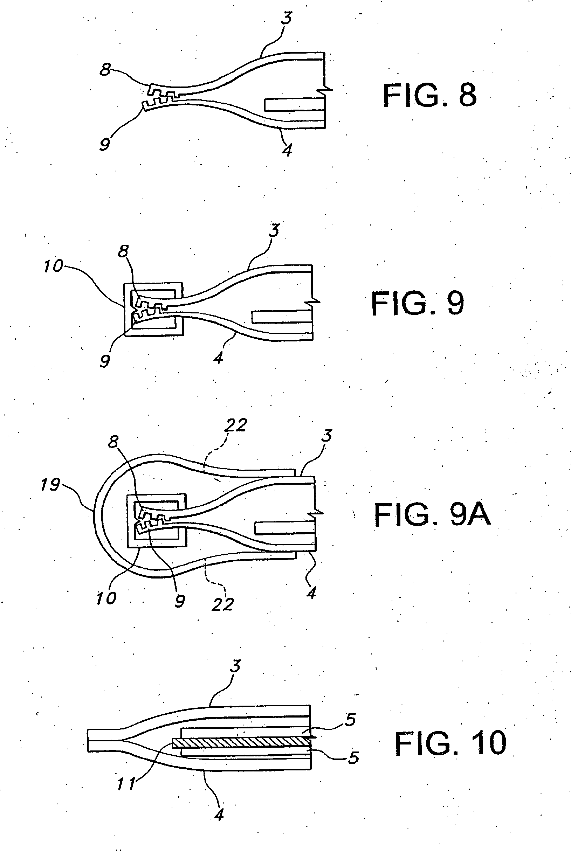

The resulting pouch may also be resealable along a portion of its perimeter as shown in FIGS. 7-9 and 9a. As shown in FIG. 7, this is accomplished by providing a portion of the perimeter with a pressure sensitive adhesive 7 between the top **3** and bottom **4** layers. At this point along the perimeter, the top and bottom layers may be separated to allow removal of a dosage form and then resealing. Alternatively, as shown in FIGS. 8 and 9, a portion of the perimeter may be sealed and resealed by forming a zipper track **8** along the top layer **3** and a corresponding track **9** along the bottom layer **4** of a portion of the pouch. The two tracks should be formed such that they can engage, forming a seal along a portion of the perimeter. The track may be engaged by either manual pressure, or by the use of a zipper **10.** FIG **9a** adds a "tamper resistant" feature to the packaging, where another layer of material **19** is included which covers the resealable zipper. This may either be attached by the use of an adhesive to the top **3** and bottom **4** layers, or alternatively may form an additional layer over the length of the top **3** and bottom **4** layers (not shown). This allows the pouch to be opened by the consumer by first removing the surrounding layer **19**, desirably at a perforated section **22** to permit access to the resealable opening.

More than one sheet of the dosage form may be included within the pouch. For example, two or more sheets may be stacked on top of each other. As shown in FIG 10, a non-water soluble support film **11**, such as a layer of polyolefin, also in the form of a sheet, may be placed between the sheets of water-soluble dosage forms to prevent the dosage **5** forms in the two or more sheets from adhering to each other.

To dispense a dosage form, the pouch is first opened. This is by either by tearing the pouch open or by separating the top and bottom layers of the pouch. The sheet may either be removed from the pouch, or desirably, where the top and bottom layers are separated, the film is presented apart from the top and bottom pouch layers, which are peeled away, to provide greater ease in dispensing. In one embodiment, the top and bottom pouch layers are peeled apart at one end to present the dosage form in an erect or vertical position for easy handling. Then, the unit sections of the film/dosage form may be separated by tearing along the weakened or perforated sections to separate a dose, or fraction thereof, from the sheet. The remainder of the sheet may then be returned to the pouch until a future dose is needed.

## Claims

1. An oral dosage delivery vehicle comprising at least one layer of a cast edible film including a uniformly distributed active ingredient, wherein said film comprises weakened sections which divide the film into segments that represent individual dosage units, and wherein said active ingredient varies no more than 10% among said dosage units.

2. The delivery vehicle of claim 1, wherein said film is self-supporting.

3. The delivery vehicle of claim 1, wherein said weakened sections are perforations.

4. The delivery vehicle of claim 1, wherein said weakened sections are scored.

5. The delivery vehicle of claim 1, wherein said active ingredient is a pharmaceutical or cosmetic active ingredient.

6. The delivery vehicle of claim 1, wherein said film includes a carrier layer of a substantially water insoluble material.

7. The delivery vehicle of claim 6, wherein said carrier layer is substantially continuous and said carrier layer includes weakened sections that correspond to the weakened sections of said film.

8. The delivery vehicle of claim 1, wherein the film has a surface with a surface area comprising voids that increase the surface area.

9. The delivery vehicle of claim 8, wherein said voids increase the speed of dissolution of said delivery vehicle.

10. The delivery vehicle of claim 8, wherein the voids are selected from the group consisting of voids that go through the entire depth of the film, voids that go through a portion of the depth of the film, and voids that are formed by casting the film on a patterned template.

11. An oral dosage delivery vehicle comprising at least one layer of a cast edible film, wherein said film comprises dosage units releasably joined by one or more weakened sections, which permit said dosage units to be detached from said film, wherein an active ingredient varies no more than 10% among said dosage units.

12. The delivery vehicle of claim 11, wherein said weakened sections contain less film composition than surrounding areas.

13. The delivery vehicle of claim 11, wherein said film is self-supporting.

14. The delivery vehicle of claim 11, wherein said weakened sections are perforations.

15. The delivery vehicle of claim 11, wherein said weakened sections are scored.

16. The delivery vehicle of claim 11, wherein said weakened sections are thinner than surrounding area.

17. The delivery vehicle of claim 12, further comprising an active ingredient, wherein said active ingredient is a pharmaceutical or cosmetic active ingredient and said active ingredient is uniformly distributed.

18. The delivery vehicle of claim 11, wherein said film includes a carrier layer of a substantially water insoluble material.

19. The delivery vehicle of claim 18, wherein said carrier layer is substantially continuous and said carrier layer includes weakened sections that correspond to the weakened sections of said film.

20. The delivery vehicle of claim 17, wherein the film has a surface with a surface area comprising voids that increase the surface area.

21. The delivery vehicle of claim 20, wherein the voids are selected from the group consisting of voids that go through the entire depth of the film, voids that go through a portion of the depth of the film, and voids that are formed by casting the film on a patterned template.

22. The delivery vehicle of claim 20, wherein said voids are uniform and maintain the uniform distribution of the active ingredient.

23. A package for the storage and dispensing of a sheet-like rapid dissolve dosage form, comprising:
a) a pouch comprising a top layer and a bottom layer each having an outer edge, wherein said top and bottom layers are sealed at the respective outer edges to define an enclosed interior surface therewithin, and b) a sheet-like rapid dissolve dosage form contained within said enclosed space; wherein said dosage form comprises at least one layer of a cast edible film comprising weakened sections which divide the film into segments that represent individual dosage units, and wherein an active ingredient varies no more than 10% among said dosage units.

24. The package of claim 23, wherein said dosage form includes an active ingredient and comprises a sheet of one or more dosage forms.

25. The package of claim 23, wherein said weakened sections include perforations.

26. The package of claim 23, wherein said weakened sections are thinner than surrounding area.

27. The package of claim 23, wherein said weakened sections form one or more equally spaced lines in a direction selected from vertical, horizontal, and combinations thereof.

28. The package of claim 23, wherein said pouch is resealable.

29. A method of storing a sheet-like rapid dissolve dosage form comprising the steps of:
a) preparing a sheet-like dosage form that includes unit doses of an active ingredient; wherein said dosage form comprises at least one layer of a cast edible film comprising weakened sections which divide the film into segments that represent individual dosage units and wherein the active ingredient varies no more than 10% among said dosage units;
b) preparing a pouch comprising top and bottom layers each having an outer edge;
c) placing said dosage form between said top and bottom layers; and
d) sealing the outer edges of said pouch.

30. A method of dispensing a sheet-like dosage form comprising the steps of:
a) preparing a sheet-like dosage form that includes an active ingredient; wherein said dosage form comprises at least one layer of a cast edible film comprising weakened sections which divide the film into segments that represent individual dosage units, and wherein the active ingredient varies no more than 10% among said dosage units;
b) preparing a pouch comprising top and bottom layers each of said layers having an edge surrounding the circumference of said layer;
c) placing said dosage form between said top and bottom layers;
d) separably sealing a portion of said edges of said pouch;
e) opening a portion of said pouch;
f) opening one or more of said sections of said dosage form along said perforations; and
g) removing said one or more dosage units from said pouch.

31. The method of claim 30, further comprising the step of resealing said pouch.

32. The method of claim 30, further comprising the step of permanently sealing the remaining portion of the edges of said pouch.

33. The delivery vehicle of claim 1, further comprising an additional layer of an edible film.

34. The delivery vehicle of claim 11, further comprising an additional layer of an edible film.

35. A pouch comprising a top layer comprising a laminate of at least two layers and a bottom layer comprising a laminate of at least two layers, wherein said pouch is sealed along its perimeter, and wherein at least one layer of an edible film, comprising weakened sections which divide the film into segments that represent individual dosage units and wherein an active ingredient varies no more than 10% among said dosage units is included in the pouch.

36. A pouch comprising a top layer and a bottom layer wherein said pouch is resealable along a portion of its perimeter, and wherein at least one layer of an edible film, comprising weakened sections which divide the film into segments that represent individual dosage units and wherein an active ingredient varies no more than 10% among said dosage units is included in the pouch.

37. The pouch of claim 36, wherein a portion of the perimeter is provided with a pressure-sensitive adhesive.

38. The pouch of claim 36, wherein a portion of the perimeter may be sealed and resealed by forming a zipper track along the top layer.

39. The pouch of claim 38, further comprising a corresponding track along the bottom layer.

## Patentansprüche

1. Darreichungsmittel zur Munddosierung umfassend zumindest eine Lage einer geprägten und verzehrbaren Folie mit einem gleichförmig verteilten Wirkstoff, wobei die Folie geschwächte Abschnitte umfasst, welche die Folie in Segmente unterteilen, welche einzelne Dosierungseinheiten repräsentieren, und wobei der Wirkstoff um nicht mehr als 10% zwischen den Dosiereinheiten variiert.

2. Darreichungsmittel gemäß Anspruch 1, wobei die Folie selbsttragend ist.

3. Darreichungsmittel gemäß Anspruch 1, wobei die geschwächten Abschnitte Perforationen sind.

4. Darreichungsmittel gemäß Anspruch 1, wobei die geschwächten Abschnitte eingekerbt sind.

5. Darreichungsmittel gemäß Anspruch 1, wobei der Wirkstoff ein pharmazeutischer oder kosmetischer Wirkstoff ist.

6. Darreichungsmittel gemäß Anspruch 1, wobei die Folie eine Trägerlage umfasst, welche aus einem im Wesentlichen wasserunlöslichen Material besteht.

7. Darreichungsmittel gemäß Anspruch 6, wobei die Trägerlage im Wesentlichen durchgängig ist und die Trägerlage geschwächte Abschnitte umfasst, welche zu den geschwächten Abschnitten der Folie korrespondieren.

8. Darreichungsmittel gemäß Anspruch 1, wobei die Folie eine Oberfläche mit einer Flächengröße aufweist, welche Hohlräume umfasst, welche die Flächengröße erhöhen.

9. Darreichungsmittel gemäß Anspruch 8, wobei die Hohlräume die Auflösungsgeschwindigkeit des Darreichungsmittels erhöhen.

10. Darreichungsmittel gemäß Anspruch 8, wobei die Hohlräume aus der Menge gewählt sind, welche aus Hohlräumen, die die Folie in ihrer gesamten Dicke durchlaufen, aus Hohlräumen, welche die Folie zu einem Teil ihrer Dicke durchlaufen, und aus Hohlräumen, welche durch Prägen der Folie auf einer Matrize ausgeprägt sind, besteht.

11. Darreichungsmittel zur Munddosierung mit zumindest einer Lage einer geprägten und verzehrbaren Folie, wobei die Folie Dosierungseinheiten umfasst, welche durch einen oder mehrere geschwächte Abschnitte lösbar miteinander verbunden sind, wobei die geschwächten Abschnitte es erlauben, die Dosierungseinheiten von der Folie abzutrennen, wobei ein Wirkstoff um nicht mehr als 10% zwischen den Dosierungseinheiten variiert.

12. Darreichungsmittel gemäß Anspruch 11, wobei die geschwächten Abschnitte weniger Folienzusammensetzung enthalten als umgebende Bereiche.

13. Darreichungsmittel gemäß Anspruch 11, wobei die Folie selbsttragend ist.

14. Darreichungsmittel gemäß Anspruch 11, wobei die geschwächten Abschnitte Perforationen sind.

15. Darreichungsmittel gemäß Anspruch 11, wobei die geschwächten Abschnitte eingekerbt sind.

16. Darreichungsmittel gemäß Anspruch 11, wobei die geschwächten Abschnitte dünner sind als umgebende Bereiche.

17. Darreichungsmittel gemäß Anspruch 12, zusätzlich mit einem Wirkstoff, wobei der Wirkstoff ein pharmazeutischer oder kosmetischer Wirkstoff ist und der Wirkstoff gleichmäßig verteilt ist.

18. Darreichungsmittel gemäß Anspruch 11, wobei die Folie eine Trägerlage aus einem im Wesentlichen wasserunlöslichen Material umfasst.

19. Darreichungsmittel gemäß Anspruch 18, wobei die Trägerlage im Wesentlichen durchgängig ist und die Trägerlage geschwächte Abschnitte umfasst, welche zu den geschwächten Abschnitten der Folie korrespondieren.

20. Darreichungsmittel gemäß Anspruch 17, wobei die Folie eine Oberfläche mit einer Flächengröße aufweist, welche Hohlräume umfasst, welche die Flächengröße erhöhen.

21. Darreichungsmittel gemäß Anspruch 20, wobei die Hohlräume aus der Menge ausgewählt sind, welche aus Hohlräumen, welche die Folie in ihrer gesamten Dicke durchlaufen, aus Hohlräumen, welche die Folie zu einem Teil ihrer Dicke durchlaufen, und aus Hohlräumen, welche durch Prägen der Folie auf einer Matrize ausgeprägt sind, besteht.

22. Darreichungsmittel gemäß Anspruch 20, wobei die Hohlräume gleichförmig sind und die gleichförmige Verteilung des Wirkstoffs bewahren.

23. Verpackung zum Aufbewahren und zur Abgabe einer blattförmigen und sich schnell auflösenden Darreichungsform, umfassend:
a) einen Beutel mit einer oberen Lage und einer unteren Lage, wobei jede davon einen äußeren Rand aufweist, und wobei die oberen und unteren Lagen an den jeweiligen äußeren Rändern verschlossen sind, um darin eine umschlossene innere Oberfläche zu definieren, und b) eine blattförmige und sich schnell auflösende Darreichungsform, welche in dem umschlossenen Raum enthalten ist; wobei die Darreichungsform zumindest eine Lage einer geprägten und verzehrbaren Folie mit geschwächten Abschnitten umfasst, welche die Folie in Segmente unterteilen, welche individuelle Dosiereinheiten repräsentieren, und wobei ein Wirkstoff um nicht mehr als 10% zwischen den Dosierungseinheiten variiert.

24. Verpackung gemäß Anspruch 23, wobei die Darreichungsform einen Wirkstoff und ein aus einer oder mehreren Darreichungsformen bestehendes Blatt umfasst.

25. Verpackung gemäß Anspruch 23, wobei die geschwächten Abschnitte Perforationen umfassen.

26. Verpackung gemäß Anspruch 23, wobei die geschwächten Abschnitte dünner sind als umgebende Bereiche.

27. Verpackung gemäß Anspruch 23, wobei die geschwächten Abschnitte eine oder mehrere gleich weit voneinander beabstandete Linien in einer Richtung bilden, welche aus vertikal, horizontal und Kombinationen davon ausgewählt sind.

28. Verpackung gemäß Anspruch 23, wobei der Beutel wiederverschließbar ist.

29. Verfahren zur Aufbewahrung einer blattförmigen und sich schnell auflösenden Darreichungsform, umfassend die folgenden Schritte:
a) Bereitstellen einer blattförmigen Darreichungsform, welche Einzeldosen eines Wirkstoffs umfasst; wobei die Darreichungsform zumindest eine Lage einer geprägten und verzehrbaren Folie mit geschwächten Abschnitten umfasst, welche die Folie in Segmente unterteilen, welche individuelle Dosierungseinheiten repräsentieren, und wobei der Wirkstoff um nicht mehr als 10% zwischen den Dosierungseinheiten variiert;
b) Bereitstellen eines Beutels mit einer oberen und unteren Schicht, wobei jede davon einen äußeren Rand aufweist;
c) Einbringen der Darreichungsform zwischen die obere und die untere Schicht; und
d) Verschließen der äußeren Ränder des Beutels.

30. Verfahren zur Abgabe einer blattförmigen Darreichungsform mit den folgenden Schritten:
a) Bereitstellen einer blattförmigen Darreichungsform, welche einen Wirkstoff umfasst, wobei die Darreichungsform zumindest eine Schicht einer geprägten und verzehrbaren Folie mit geschwächten Abschnitten umfasst, welche die Folie in Segmente unterteilen, welche individuelle Dosierungseinheiten repräsentieren, und wobei der Wirkstoff um nicht mehr als 10% zwischen den Dosierungseinheiten variiert;
b) Bereitstellen eines Beutels mit einer oberen und unteren Schicht, wobei jede dieser Schichten einen Rand aufweist, welcher den Umfang der jeweiligen Schicht umgibt;
c) Einbringen der Darreichungsform zwischen die obere und die untere Schicht;
d) separates Verschließen eines Abschnitts der Ränder des Beutels;
e) Öffnen eines Abschnitts des Beutels;
f) Auftrennen einer oder mehrerer der geschwächten Abschnitte der Darreichungsform entlang der Perforationen; und
g) Entfernen der einen oder mehreren Dosierungseinheiten aus dem Beutel.

31. Verfahren gemäß Anspruch 30, zusätzlich umfassend den Schritt des Wiederverschließens des Beutels.

32. Verfahren gemäß Anspruch 30, zusätzlich umfassend den Schritt des dauerhaften Verschließens des verbleibenden Abschnitts der Ränder des Beutels.

33. Darreichungsmittel gemäß Anspruch 1, zusätzlich umfassend eine weitere Schicht einer verzehrbaren Folie.

34. Darreichungsmittel gemäß Anspruch 11, zusätzlich umfassend eine weitere Schicht einer verzehrbaren Folie.

35. Beutel mit einer oberen Schicht, welche ein Laminat aus zumindest zwei Schichten umfasst, und einer unteren Schicht, welche ein Laminat aus zumindest zwei Schichten umfasst, wobei der Beutel entlang seines Umfangs verschlossen ist, und wobei zumindest eine Lage einer verzehrbaren Folie mit geschwächten Abschnitten, welche die Folie in Segmente unterteilen, welche individuelle Dosierungseinheiten repräsentieren, und wobei der Wirkstoff um nicht mehr als 10% zwischen den Dosierungseinheiten variiert, in dem Beutel enthalten ist.

36. Beutel mit einer oberen und einer unteren Schicht, wobei der Beutel entlang eines Abschnitts seines Umfangs wiederverschließbar ist, und wobei zumindest eine Lage einer verzehrbaren Folie mit geschwächten Abschnitten, welche die Folie in Segmente unterteilen, welche individuelle Dosierungseinheiten repräsentieren, und wobei der Wirkstoff um nicht mehr als 10% zwischen den Dosierungseinheiten variiert, in dem Beutel enthalten ist.

37. Beutel gemäß Anspruch 36, wobei ein Abschnitt des Umfangs mit einem druckempfindlichen Kleber ausgestattet ist.

38. Beutel gemäß Anspruch 36, wobei ein Abschnitt des Umfangs durch Ausbildung einer Reißverschlussbahn entlang der oberen Schicht verschließbar und wiederverschließbar ist.

39. Beutel gemäß Anspruch 38, zusätzlich umfassend eine entsprechende Bahn entlang der unteren Lage.

## Revendications

1. Véhicule d'administration de forme de dose orale comprenant au moins une couche d'un film comestible coulé comprenant une substance active uniformément distribuée, ledit film comprenant des sections affaiblies qui divisent le film en segments qui représentent des doses unitaires individuelles, et ladite substance active ne variant pas de plus de 10 % entre lesdites doses unitaires.

2. Véhicule d'administration de la revendication 1, dans lequel ledit film est autosoutenu.

3. Véhicule d'administration de la revendication 1, dans lequel lesdites sections affaiblies sont des perforations.

4. Véhicule d'administration de la revendication 1, dans lequel lesdites sections affaiblies sont sécables.

5. Véhicule d'administration de la revendication 1, dans lequel ladite substance active est une substance active pharmaceutique ou cosmétique.

6. Véhicule d'administration de la revendication 1, dans lequel ledit film comprend une couche de support d'un matériau sensiblement insoluble dans l'eau.

7. Véhicule d'administration de la revendication 6, dans lequel ladite couche de support est sensiblement continue et ladite couche de support comprend des sections affaiblies qui correspondent aux sections affaiblies dudit film.

8. Véhicule d'administration de la revendication 1, dans lequel le film a une surface comprenant des vides qui augmentent la surface.

9. Véhicule d'administration de la revendication 8, dans lequel lesdits vides augmentent la vitesse de dissolution dudit véhicule d'administration.

10. Véhicule d'administration de la revendication 8, dans lequel les vides sont choisis dans le groupe constitué de vides qui traversent la profondeur totale du film, de vides qui traversent une partie de la profondeur du film, et de vides qui sont formés par coulée du film sur une matrice modelée.

11. Véhicule d'administration de forme de dose orale comprenant au moins une couche d'un film comestible coulé, ledit film comprenant des unités de dose assemblées de façon détachable par une ou plusieurs sections affaiblies, qui permettent que lesdites doses unitaires soient détachées dudit film, une substance active ne variant pas de plus de 10 % entre lesdites unités de dose.

12. Véhicule d'administration de la revendication 11, dans lequel lesdites sections affaiblies contiennent moins de composition de film que les zones environnantes.

13. Véhicule d'administration de la revendication 11, dans lequel ledit film est autosoutenu.

14. Véhicule d'administration de la revendication 11, dans lequel lesdites sections affaiblies sont des perforations.

15. Véhicule d'administration de la revendication 11, dans lequel lesdites sections affaiblies sont sécables.

16. Véhicule d'administration de la revendication 11, dans lequel lesdites sections affaiblies sont plus minces que la zone environnante.

17. Véhicule d'administration de la revendication 12, comprenant en outre une substance active, dans lequel ladite substance active est une substance active pharmaceutique ou cosmétique et ladite substance active est uniformément distribuée.

18. Véhicule d'administration de la revendication 11, dans lequel ledit film comprend une couche de support d'un matériau sensiblement insoluble dans l'eau.

19. Véhicule d'administration de la revendication 18, dans lequel ladite couche de support est sensiblement continue et ladite couche de support comprend des sections affaiblies qui correspondent aux sections affaiblies dudit film.

20. Véhicule d'administration de la revendication 17, dans lequel le film a une surface avec une surface comprenant des vides qui augmentent la surface.

21. Véhicule d'administration de la revendication 20, dans lequel les vides sont choisis dans le groupe constitué de vides qui traversent la profondeur totale du film, de vides qui traversent une partie de la profondeur du film, et de vides qui sont formés par coulée du film sur une matrice modelée.

22. Véhicule d'administration de la revendication 20, dans lequel lesdits vides sont uniformes et maintiennent la distribution uniforme de la substance active.

23. Emballage pour le stockage et la distribution d'une forme de dose à dissolution rapide en forme de feuille, comprenant:
a) une poche comprenant une couche supérieure et une couche inférieure ayant un bord externe, lesdites couches supérieure et inférieure étant scellées aux bords externes respectifs pour définir une surface intérieure fermée à l'intérieur de celles-ci, et b) une forme dose à dissolution rapide en forme de feuille contenue à l'intérieur dudit espace fermé ; ladite forme de dose comprenant au moins une couche d'un film comestible coulé comprenant des sections affaiblies qui divisent le film en segments qui représentent des doses unitaires individuelles, et une substance active ne variant pas de plus de 10 % entre lesdites unités de dose.

24. Emballage de la revendication 23, dans lequel ladite forme de dose comprend une substance active et comprend une feuille d'une ou plusieurs formes de dose.

25. Emballage de la revendication 23, dans lequel lesdites sections affaiblies comprennent des perforations.

26. Emballage de la revendication 23, dans lequel lesdites sections affaiblies sont plus minces que la zone environnante.

27. Emballage de la revendication 23, dans lequel lesdites sections affaiblies forment une ou plusieurs lignes également espacées dans une direction choisie parmi les directions verticale, horizontale et des combinaisons de celles-ci.

28. Emballage de la revendication 23, dans lequel ladite poche est détachable.

29. Procédé de stockage d'une forme de dose à dissolution rapide de type feuille comprenant les étapes de :
a) préparation d'une forme de dose de type feuille qui comprend des doses unitaires d'une substance active ;
ladite forme de dose comprenant au moins une couche d'un film comestible coulé comprenant des sections affaiblies qui divisent le film en segments qui représentent des doses unitaires individuelles, et la substance active ne variant pas de plus de 10 % entre lesdites unités de dose ;
b) préparation d'une poche comprenant des couches supérieure et inférieure ayant chacune un bord externe ;
c) placement de ladite forme de dose entre lesdites couches supérieure et inférieure ; et
d) scellement des bords externes de ladite poche.

30. Procédé de distribution d'une forme de dose de type feuille comprenant les étapes de :
a) préparation d'une forme de dose de type feuille qui comprend une substance active ;
ladite forme de dose comprenant au moins une couche d'un film comestible coulé comprenant des sections affaiblies qui divisent le film en segments qui représentent des doses unitaires individuelles, et la substance active ne variant pas de plus de 10 % entre lesdites unités de dose ;
b) préparation d'une poche comprenant des couches supérieure et inférieure ayant un bord entourant la circonférence de ladite couche ;
c) placement de ladite forme de dose entre lesdites couches supérieure et inférieure ;
d) scellement de façon séparable d'une partie desdits bords de ladite poche ;
e) ouverture d'une partie de ladite poche ;
f) ouverture d'une ou plusieurs desdites sections de ladite forme de dose le long desdites perforations ; et
g) retrait desdites une ou plusieurs unités de dose depuis ladite poche.

31. Procédé de la revendication 30, comprenant en outre l'étape de rescellement de ladite poche.

32. Procédé de la revendication 30, comprenant en outre l'étape de scellement permanent de la partie résiduelle des bords de ladite poche.

33. Véhicule d'administration de la revendication 1, comprenant en outre une couche additionnelle d'un film comestible.

34. Véhicule d'administration de la revendication 11, comprenant en outre une couche additionnelle d'un film comestible.

35. Poche comprenant une couche supérieure comprenant un stratifié d'au moins deux couches et une couche inférieure comprenant un stratifié d'au moins deux couches, ladite poche étant scellée le long de son périmètre, et au moins une couche d'un film comestible, comprenant des sections affaiblies qui divisent le film en segments qui représentent des doses unitaires individuelles et la substance active ne variant pas de plus de 10 % entre lesdites unités de dose, étant incluse dans la poche.

36. Poche comprenant une couche supérieure et une couche inférieure, ladite poche étant détachable le long d'une partie de son périmètre, et au moins une couche d'un film comestible, comprenant des sections affaiblies qui divisent le film en segments qui représentent des doses unitaires individuelles et la substance active ne variant pas de plus de 10 % entre lesdites unités de dose, étant incluse dans la poche.

37. Poche de la revendication 36, dans laquelle une partie du périmètre est pourvue d'un adhésif autocollant.

38. Poche de la revendication 36, dans laquelle une partie du périmètre peut être scellée et rescellée en formation une piste de fermeture à glissière.

39. Poche de la revendication 38, comprenant en outre une piste correspondante le long de la couche inférieure.
